Europäisches Patentamt

European Patent Office

Office européen des brevets

(19)

(11) Veröffentlichungsnummer: **0 450 262 A1**

# (12) EUROPÄISCHE PATENTANMELDUNG

(21) Anmeldenummer: 90890337.0

(22) Anmeldetag: 18.12.90

(51) Int. Cl.⁵: **A61K 9/22, A61K 9/34**

(30) Priorität: 04.04.90 AT 803/90

(43) Veröffentlichungstag der Anmeldung:
09.10.91 Patentblatt 91/41

(84) Benannte Vertragsstaaten:
AT BE CH DE DK ES FR GB GR IT LI LU NL SE

(71) Anmelder: LENZING AKTIENGESELLSCHAFT
Werkstrasse 1
A-4860 Lenzing(AT)

(72) Erfinder: Korsatko-Wabnegg, Brigitta, Mag.Dr.
Kärtnerstrasse 432
A-8054 Graz(AT)
Erfinder: Korsatko, Werner, Dr.
Kärtnerstrasse 432
A-8054 Graz(AT)
Erfinder: Wegleitner, Karlheinz, Dipl.-Ing., Dr.
Dr.-Adolf-Schärf-Strasse 12 / Tür 1
A-4040 Linz(AT)

(74) Vertreter: Müllner, Erwin, Dr. et al
Patentanwälte, Dr. Erwin Müllner, Dipl.-Ing.
Werner Katschinka, Dr. Martin Müllner,
Postfach 159, Weihburggasse 9
A-1010 Wien(AT)

(54) **Pharmazeutisches Präparat mit verzögerter Wirkstofffreisetzung.**

(57) Pharmazeutisches Präparat mit verzögerter Wirkstofffreisetzung ("delayed release"), das neben dem Wirkstoff oder den Wirkstoffen Hemicellulose in Kombination mit einem nicht zerfallenden, matrixbildenden Hilfsstoff enthält. Vorzugsweise ist es als Manteltablette oder -dragee ausgebildet, wobei der Kern den Wirkstoff oder die Wirkstoffe enthält und der Mantel die Hemicellulose und den Hilfsstoff enthält. Als Hilfsstoff kommt Ethylcellulose, Eudragit RS PM, Poly-(L-lactid), Poly-(D,L-lactidcoglycolid) und Poly-D(-)-3-hydroxybutyrat-co-hydroxyvalerat in Frage.

EP 0 450 262 A1

Die vorliegende Erfindung betrifft ein pharmazeutisches Präparat mit verzögerter Wirkstofffreisetzung ("delayed release").

Es ist bekannt, daß zur Herstellung von Tabletten für die perorale Applikation pharmazeutische Wirkstoffe vor dem Verpressen häufig mit verschiedenen, in der Galenik üblichen Hilfs- und Zusatzstoffen vermischt werden müssen, wobei jedoch die Eignung dieser Hilfsstoffe, etwa bezüglich ihrer Komprimierungseigenschaften, nicht immer völlig zufriedenstellend ist. Beispiele für Tablettenhilfsstoffe dieser Art sind u.a. im Lehrbuch der pharmazeutischen Technologie, R. Voigt, 5.Aufl., Verlag Chemie, Weinheim (1984), S. 178 ff. beschrieben und umfassen Substanzen wie Gleitmittel, Bindemittel, Füllmittel, Fließregulierungsmittel, Schmiermittel, Zerfallsmittel, Feuchthaltemittel, Adsorptionsmittel, etc.

Weiters ist bekannt, daß zur Herstellung solcher Tabletten häufig ein Granuliervorgang notwendig ist, um eine entsprechende Rieselfähigkeit und Schüttdichte der Pulvermischung zu gewährleisten. Diese Parameter spielen eine wichtige Rolle für eine problemlose Tablettierung im großtechnischen Maßstab. Der Granuliervorgang ist allerdings meist aufwendig, man benötigt dazu beispielsweise Polymere unterschiedlichster Natur, welche als Klebstoffe fungieren und eine Aggregation der Pulverpartikel bewirken. Wenn man bedenkt, daß in der Literatur Tablettenrezepturen mit bis zu 20 verschiedenen Bestandteilen angeführt werden, wird verständlich, daß das Auftreten von Wechselwirkungen zwischen Wirkstoffen und Hilfsstoffen nicht auszuschließen ist. Es hat daher nicht an Versuchen gefehlt, Tablettenhilfsstoffe zu finden, die mehrere der oben genannten Eigenschaften in sich vereinen, so daß die Anzahl der Hilfsstoffe reduziert werden bzw. gegebenenfalls eine Granulierung unterlassen werden kann. Beispiele für Tablettenhilfsstoffe dieser Art sind mikrokristalline Cellulose (Avicel, Heweten) und sprühgetrocknete Lactose vor allem für die Brikettierung und Direkttablettierung. Aber auch eine Hemicellulose, nämlich Xylan, wurde bereits als Füllstoff und Sprengmittel für Tabletten vorgeschlagen. (S. M.Juslin, P.Paronen: Xylan - a possible filler and disintegrant for tablets", J.Pharm.Pharmacol. 1984, 36; 256-257). Es wird gemäß dieser Arbeit Hemicellulose zur Herstellung von verpreßten Arzneiformen verwendet, aus denen im Anwendungsfall der Wirkstoff sofort, d.h. innerhalb weniger Sekunden oder höchstens Minuten, freigesetzt wird. Hemicellulose besitzt gute Verpressungseigenschaften und bildet ein kohärentes Tablettengerüst aus. Der Zusatz von Zerfallsmitteln kann unterbleiben.

Es wurde nun überraschend gefunden, daß Hemicellulose auch hervorragend zur Herstellung von "delayed-release"-Präparaten verwendet werden kann.

Gegenstand der Erfindung ist daher ein pharmazeutisches Präparat mit verzögerter Wirkstofffreisetzung ("delayed release"), das erfindungsgemäß neben dem Wirkstoff oder den Wirkstoffen Hemicellulose in Kombination mit einem nicht zerfallenden, matrixbildenden Hilfsstoff enthält.

Hemicellulose besitzt nämlich ein konstantes Quellverhalten, welches sich direkt proportional zur Zeit verhält. Diese Eigenschaft ermöglicht einerseits eine Einstellung der Zerfallszeit des Preßlings und daher eine vorausberechenbare Verzögerung der Wirkstofffreigabe aus entsprechenden Tabletten im Sinne eines "delayed-release"-Effektes.

Es ist eine besonders hervorzuhebende Eigenschaft der erfindungsgemäß eingesetzten Hemicellulose, daß der Zerfall der Preßlinge unabhängig vom pH-Wert des Liberationsmediums (Gastro-Intestinaltrakt) erfolgt.

Nicht zerfallende matrixbildende Hilfsstoffe können also durch die Kombination mit der erfindungsgemäßen Hemicellulose aufgrund deren reproduzierbarer Quellfähigkeit zur Herstellung von zeitlich definiert zerfallenden Preßlingen eingesetzt werden. Der Zeitpunkt des Zerfalls solcher Tabletten wird durch die Art des matrixbildenden Hilfsstoffes und die Konzentration der erfindungsgemäß eingesetzten Hemicellulose bestimmt und bewegt sich in einem Bereich von 1 bis 8 Stunden.

Ein besonderes Einsatzgebiet solcher Kombinationen von nichtzerfallenden matrixbildenden Hilfsstoffen und Hemicellulose ist die Herstellung von Manteltabletten mit "delayed-release"-Effekt. Gegenstand der Erfindung ist daher auch ein pharmazeutisches Präparat, das als Manteltablette oder -dragee ausgebildet ist, wobei der Kern den Wirkstoff oder die Wirkstoffe enthält und der Mantel die Hemicellulose und den Hilfsstoff enthält. Dadurch wird der Wirkstoff aus dem Tablettenkern in Abhängigkeit von der Konzentration an Hemicellulose im Mantel erst zu einem vorausberechneten Zeitpunkt pH-unabhängig freigegeben, sodaß dieses System eine gezielte Steuerung des Wirkungseintrittes ermöglicht. Durch die Möglichkeit, den Tablettenkern rasch zerfallend oder retardierend zu gestalten, eröffnet sich eine Vielzahl von Einsatzgebieten.

Manteltabletten oder -dragees dieser Art sind insbesondere für Patienten mit chronischem Krankheitsbild von Bedeutung und stellen sowohl für den Patienten als auch für Arzt und Pflegepersonal eine nicht zu unterschätzende Entlastung dar. Speziell in jenen Fällen, in denen der Arzneistoff nach Applikation der "delayed-release"-Formulierung vor dem Schlafengehen seine Wirkung unabhängig davon, in welchem Abschnitt des Gastro-Intestinaltraktes sich die Manteltablette befindet, erst am frühen Morgen entfaltet, wird die Lebenssituation dieser Patienten entscheidend verbessert.

Als nicht zerfallende matrixbildende Hilfsstoffe sind dabei geeignet (in Klammer ist jeweils das Verhältnis von Hemicellulose zu Hilfsstoff angegeben):

a) Ethylcellulose (35:65 - 15:85)

b) Eudragit RS PM ( 5:95 - 25:75)

c) Poly-(L-lactid) (35:65 - 15:85)

d) Poly-(D,L-lactid-coglycolid) (40:60 - 15:85)

e) Poly-D(-)3-hydroxybutyrat-co-hydroxyvalerat (5:95 - 20:80).

Es ist bevorzugt, daß die Hemicellulose einen DP zwischen 10 und 250, vorzugsweise 50, aufweist, daß sie ein Schüttvolumen von 1,3 bis 1,5 ml.g$^{-1}$ besitzt, daß sie eine Korngröße von 2 bis 50$\mu$m, vorzugsweise 10 bis 20$\mu$m umfaßt, daß sie von sphärischer Gestalt ist und daß sie eine Sauggeschwindigkeit nach B.M.Lichstein von maximal 0,3 ml/s aufweist. (Bernard M.Lichstein, Johnson u.Johnson: "Demand Wettability and new methods of measuring absorbency characteristics of Gabic", 2.Annual Symp. on Nonwovens Product Development 1974 wash D.C.)

Diese aus Laubholzsulfitzellstoff gewonnene Hemicellulose besteht zu 90% aus Xylan mit einem Molekulargewicht von 5300. Die restlichen 10% setzen sich aus Glucan und Mannan zusammen. Das Molekulargewicht und die Zusammensetzung lassen erkennen, daß es sich bei diesem Produkt um eine bereits stark abgebaute Hemicellulose handelt. Aufgrund ihrer Zusammensetzung bildet die wasserunlösliche Substanz kein Hydrokolloid wie Stärke, Pektin oder CMC, besitzt aber eine Saugkapazität von 2,8 ml.g$^{-1}$, woraus eine gute Sprengmittelwirkung resultiert. Die sphärische Gestalt und die Partikelgröße bedingen ein gleichmäßiges Quellverhalten in wässerigem Milieu. Als weiteres Merkmal der Hemicellulose ist hervorzuheben, daß die Quellung zusätzlich pH-unabhängig erfolgt.

Diese besonderen Eigenschaften ermöglichen bei den hier vorgestellten Manteltabletten eine zeitlich definierbare Aufsprengung und somit eine exakt vorausberechenbare Verzögerung der Wirkstofffreigabe im Sinne eines "delayed-release"-Effektes, wobei die pH-Verhältnisse des Liberationsmediums (Gastrointestinaltrakt) im Vergleich zu anderen Systemen mit zeitlich verzögerter Wirkstofffreigabe, wie magensaftresistent überzogene Tabletten, keinen Einfluß auf den vorprogrammierten Liberationseintritt ausüben.

Hemicellulose wird durch Dickdarmbakterien des menschlichen Organismus teilweise fermentiert, die Abbauquote liegt zwischen 40% und 60%. Daher kann es durch eventuell im Organismus verbleibende Tablettenreste nicht zu einer Akkumulation kommen, wie das bei Hilfsstoffen auf unverdaulicher Polymerbasis der Fall sein könnte.

Die nachfolgenden Beispiele sollen das Wesen der Erfindung erläutern.

Beispiel 1

Bestimmung der Zerfallszeit von Hemicellulose/Ethylcellulose-Komprimaten in Abhängigkeit zu Mischungsverhältnis und pH-Wert.

Die Bestandteile wurden homogen vermischt und ohne Vorgranulierung mittels einer elektrohydraulischen Presse unter einem Druck von 98,1 N pro Tablette zu Tabletten verpreßt. Das Durchschnittsgewicht der Tabletten betrug 100 ± 3 mg, der Durchmesser der Tabletten 7 mm, die Höhe etwa 2 mm. Die Messung der Zerfallszeiten der Tabletten wurde nach der im Europäischen Arzneibuch, Bd. III (1981), s. 78 ff geoffenbarten Methode bei verschiedenen pH-Werten vorgenommen: sie sind in Tabelle 1 zusammengefaßt. Es konnte keine pH-Abhängigkeit nachgewiesen werden.

Tabelle 1

| Mischungsverhältnis (%) (Hemicellulose/Ethylcellulose) | Zerfallszeit (Minuten) | |
|---|---|---|
| | pH 1 | pH 7 |
| 50 / 50 | 29,1 | 31,7 |
| 40 / 60 | 41,0 | 41,5 |
| 30 / 70 | 96,0 | 95,0 |
| 20 / 80 | 330,0 | 330,0 |
| 10 / 90 | 440,0 | 420,0 |

Beispiel 2

Bestimmung der Zerfallszeit von Preßlingen bestehend aus Hemicellulose und verschiedenen nichtzerfallenden matrixbildenden Hilfsstoffen in Abhängigkeit zum pH-Wert. Hemicellulose wurde mit Eudragit RS PM, mit Ethylcellulose sowie mit Poly-(L-lactid) jeweils in einem Gewichtsverhältnis 1 : 1 homogen vermischt und analog Beispiel 1 zu Tabletten verpreßt.
Die Ergebnisse des Zerfallstestes (Tabelle 2) zeigen keine pH-Abhängigkeit.

Tabelle 2

| pH-Wert | Zerfallszeit (Minuten) | | |
|---|---|---|---|
| | Hemicellulose/ Eudragit RS PM ( 1 : 1) | Hemicellulose/ Ethylcellulose (1 : 1) | Hemicellulose/ Poly-(L-lactid) (1 : 1) |
| 1 | 5,3 | 29,1 | 24,5 |
| 2 | 5,6 | 29,8 | 24,3 |
| 3 | 5,7 | 30,0 | 24,8 |
| 4 | 5,8 | 30,0 | 26,0 |
| 5 | 6,1 | 30,6 | 26,7 |
| 6 | 6,1 | 30,9 | 27,8 |
| 7 | 6,6 | 31,7 | 31,2 |

Beispiel 3:

Bestimmung der Zerfallszeit von Hemicellulose/Eudragit RS PM - Komprimaten in Abhängigkeit zu Mischungsverhältnis und pH-Wert.
Aus verschiedenen Gewichtsanteilen Hemicellulose und Eudragit RS PM im Bereich von 90:10 bis 10:90 wurden analog Beispiel 1 Tabletten hergestellt und dem Zerfallstest unterworfen.
Die Ergebnisse (Tabelle 3) zeigen keine pH-Abhängigkeit.

Tabelle 3

| Mischungsverhältnis (%) Hemicellulose/Eudragit RS PM | Zerfallszeit (Minuten) | | |
|---|---|---|---|
| | pH 1 | pH 4 | pH 7 |
| 90 / 10 | 9,1 | 11,4 | 12,0 |
| 80 / 20 | 8,1 | 10,1 | 10,6 |
| 70 / 30 | 5,9 | 7,7 | 6,9 |
| 60 / 40 | 5,2 | 6,3 | 6,5 |
| 50 / 50 | 5,3 | 5,6 | 6,3 |
| 40 / 60 | 2,2 | 2,7 | 2,6 |
| 30 / 70 | 1,0 | 1,0 | 1,0 |
| 20 / 80 | 2,5 | 3,0 | 3,0 |
| 10 / 90 | 106,6 | 113,2 | 145,0 |

Beispiel 4

Herstellung von Manteltabletten mit rasch zerfallenden Tabletenkernen.

Tablettenkerne:  A)   5 mg 7-Hydroxyethyltheophyllin

6 mg Ethylcellulose

14 mg Hemicellulose

B)   5 mg 7-Hydroxyethyltheophyllin

20 mg Granulatum simplex (Lehrbuch der

pharmazeutischen Technologie, R.

Voigt, S.178)

C)   5 mg 7-Hydroxyethyltheophyllin

19 mg Granulatum simplex

1 mg Hemicellulose

Die Bestandteile wurden homogen vermischt und mit einem Druck von 49,05 N/Tablette zu Tabletten mit einem Durchschnittsgewicht von 25 ± 0,3 mg, einem Durchmesser von 4 mm und einer Höhe von 1,2 mm verpreßt.

Tablettenmantel:  I)   157,5 mg Ethylcellulose 67,5 mg Hemicellulose

II)   180,0 mg Ethylcellulose

45,0 mg Hemicellulose

Herstellung der Manteltabletten: Die Umhüllung der Tablettenkerne A, B und C erfolgte durch Aufpressen der mantelbildenden Hilfsstoffmischungen I und II auf den Tablettenkern.

Maße der Manteltabletten: Gewicht:      250 mg ± 1,5 mg

Durchmesser:    9,0 mm

Höhe:        4,5 mm

Die Überprüfung des "delayed-release"-Effektes erfolgte nach der "half-change" Methode (R.Voigt, Lehrbuch der pharmazeutischen Technologie, S. 627) durch spektralphotometrische Erfassung des freigesetzten 7-Hydroxyethyltheophyllins bei 273 nm. Die Ergebnisse sind aus Tabelle 4 für den Tablettenmantel I und aus Tabelle 5 für den Tablettenmantel II ersichtlich.

Tabelle 4

| Stunden | Wirkstofffreigabe aus dem Tablettenkern (%) | | |
|---|---|---|---|
| | A | B | C |
| 1 | - | - | - |
| 2 | 28,9 | 20,0 | 9,6 |
| 3 | 98,3 | 91,7 | 81,7 |
| 4 | 100,0 | 94,5 | 89,1 |
| 5 | 100,0 | 100,0 | 100,0 |

Verzögerung des Wirkungseintrittes: 2 Stunden.

Tabelle 5

| Stunden | Wirkstofffreigabe aus dem Tablettenkern (%) | | |
|---|---|---|---|
| | A | B | C |
| 6 | - | - | - |
| 7 | 70,3 | - | - |
| 8 | 100,0 | 64,5 | 58,8 |
| 9 | 100,0 | 95,3 | 92,0 |
| 10 | 100,0 | 100,0 | 100,0 |

Verzögerung des Wirkungseintritts: 7 bis 7,5 Stunden.

Beispiel 5

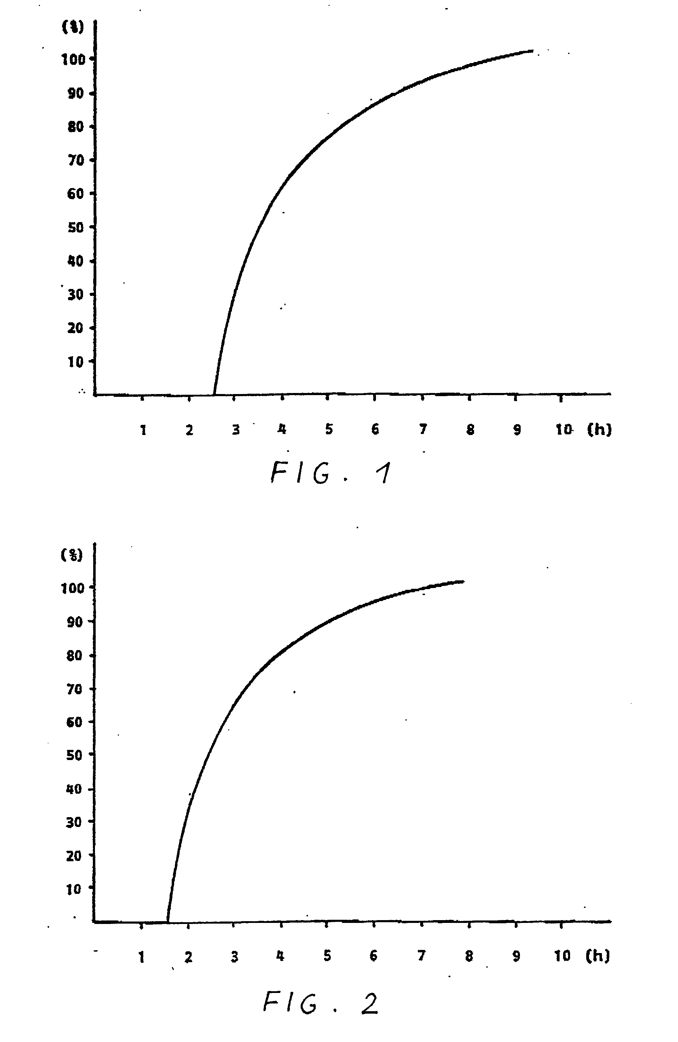

Herstellung von Manteltabletten mit retardierendem Tablettenkern.
Tablettenkern:     5 mg 7-Hydroxyethyltheophyllin 20 mg Ethylcellulose
Die Herstellung des Tablettenkernes erfolgte analog Beispiel 4.
Tablettenmantel:     157,5 mg Ethylcellulose 67,5 mg Hemicellulose
Die Herstellung und Überprüfung der Manteltabletten erfolgte analog Beispiel 4. Die Ergebnisse sind in Fig. 1 und in Tabelle 6 dargestellt.

Tabelle 6

| Stunden | Wirkstofffreigabe aus dem Tablettenkern (%) |
|---|---|
| 2 | - |
| 3 | 30,9 |
| 4 | 77,4 |
| 6 | 88,9 |
| 7 | 92,8 |
| 8 | 96,1 |
| 9 | 100,0 |

Verzögerung des Wirkungseintrittes: 2,5 Stunden.

Beispiel 6

Herstellung von Manteltabletten mit retardierendem Tablettenkern.
Tablettenkern:     5 mg 7-Hydroxyethyltheophyllin 20 mg Ethylcellulose
Die Herstellung des Tablettenkernes erfolgte analog Beispiel 4.
Tablettenmantel:     157,5 mg Poly-(L-lactid) 67,5 mg Hemicellulose
Die Herstellung und Überprüfung der Manteltabletten erfolgte analog Beispiel 4. Die Ergebnisse sind aus
Fig. 2 und aus Tabelle 7 ersichtlich.

Tabelle 7

| Stunden | Wirkstofffreigabe aus dem Tablettenkern (%) |
|---------|---------------------------------------------|
| 1 | – |
| 2 | 38,8 |
| 3 | 68,2 |
| 4 | 80,4 |
| 5 | 89,7 |
| 6 | 94,1 |
| 7 | 97,8 |
| 8 | 100,0 |

Verzögerung des Wirkungseintritts: 1,5 Stunden.

**Patentansprüche**

1. Pharmazeutisches Präparat mit verzögerter Wirkstofffreisetzung ("delayed release"), dadurch gekennzeichnet, daß es neben dem Wirkstoff oder den Wirkstoffen Hemicellulose in Kombination mit einem nicht zerfallenden, matrixbildenden Hilfsstoff enthält.

2. Pharmazeutisches Präparat nach Anspruch 1, dadurch gekennzeichnet, daß es als Manteltablette oder -dragee ausgebildet ist, wobei der Kern den Wirkstoff oder die Wirkstoffe enthält und der Mantel die Hemizellulose und den Hilfsstoff enthält.

3. Pharmazeutisches Präparat nach Anspruch 2, dadurch gekennzeichnet, daß der Hilfsstoff Ethylcellulose ist und daß das Verhältnis der Hemicellulose zur Ethylcellulose im Bereich von 35 : 65 bis 15 : 85 liegt.

4. Pharmazeutisches Präparat nach Anspruch 2, dadurch gekennzeichnet, daß der Hilfsstoff Eudragit RS PM ist und daß das Verhältnis der Hemicellulose zum Eudragit RS PM im Bereich von 5 : 95 bis 25 : 75 liegt.

5. Pharmazeutisches Präparat nach Anspruch 2, dadurch gekennzeichnet, daß der Hilfsstoff Poly-(L-lactid) ist und daß das Verhältnis der Hemicellulose zum Poly-(L-lactid) im Bereich von 35 : 65 bis 15 : 85 liegt.

6. Pharmazeutisches Präparat nach Anspruch 2, dadurch gekennzeichnet, daß der Hilfsstoff Poly-(D,L-lactid-coglycolid) ist und daß das Verhältnis der Hemicellulose zum Poly-(D,L-lactidcoglycolid) im Bereich von 40 : 60 bis 15 : 85 liegt.

7. Manteltablette oder -dragee nach Anspruch 2, dadurch gekennzeichnet, daß der Hilfsstoff Poly-D(-)-3-hydroxybutyrat-cohydroxyvalerat ist und daß das Verhältnis der Hemicellulose zum Poly-D(-)-3-hydroxybutyrat-co-hydroxyvalerat im Bereich von 5 : 95 bis 20 : 80 liegt.

8. Manteltablette oder -dragee nach einem der Ansprüche 1 bis 7, dadurch gekennzeichnet, daß die Hemicellulose einen DP zwischen 10 und 250, vorzugsweise 50, aufweist, daß sie ein Schüttvolumen von 1,3 bis 1,5 ml.g$^{-1}$ besitzt, daß sie eine Korngröße von 2 bis 50 $\mu$m, vorzugsweise 10 bis 20 $\mu$m umfaßt, daß sie von sphärischer Gestalt ist und daß sie eine Sauggeschwindigkeit nach B.H. Lichstein von maximal 0,3 ml/s aufweist.

FIG . 1

FIG . 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 105, Nr. 23, 8. Dezember 1986, Seite 502, Zusammenfassung Nr. 207827g, Columbus, Ohio, US; <br> & SU-A-1 250 240 (ODESSA TECHNOLOGICAL INSTITUTE OF THE FOOD INDUSTRY) 15-08-1986 <br> * Zusammenfassung * <br> — — — | 1 | A 61 K 9/22 <br> A 61 K 9/34 |
| Y | EP-A-0 272 119 (SYNTEX INC.) <br> * Seite 2, Zeilen 23-37; Seite 3, Zeilen 45-53; Seite 4, Zeilen 7-9; Seite 5, Beispiel 1(A); Ansprüche 15,18-20 * <br> — — — | 1-7 | |
| Y | WO-A-8 904 673 (BOARD OF REGENTS OF THE UNIVERSITY OF TEXAS SYSTEM) <br> * Seite 1, Zeilen 3-7; Seite 2, Zeilen 16-26; Seite 5, Zeile 6 - Seite 6, Zeile 26; Seiten 13-15, Beispiele 2,3; Ansprüche 1,2,8 * <br> — — — | 1,2,5-7 | |
| X | DERWENT FILE SUPPLIER WPI, 1980, AN = 80-37514C [21], Derwent Publications Ltd, Londen, GB; <br> & JP-A-55 051 024 (H. IIDA) <br> * Zusammenfassung * <br> — — — | 3 | |
| Y | EUDRAGIT DATENBLATT, RÖHM PHARMA GmbH, 1983; "Eudragit RL/RS PM" <br> * Die Anwendungsmöglichkeiten; die Verarbeitung * <br> — — — | 4 | RECHERCHIERTE SACHGEBIETE (Int. Cl.5) <br><br> A 61 K |
| P,X | EP-A-0 371 493 (NISSHIN FLOUR MILLING CO., LTD) <br> * Seite 2, Zeilen 6-10; Seite 4, Zeilen 33-34; Seite 5, Zeilen 17-22; Seiten 9,10, Beispiel 7; Anspruch 4 * <br> — — — | 1 | |
| Y | WO-A-8 706 241 (CARBOMATRIX AB) <br> * Seite 1, Zeilen 1-5; Seite 5, Zeilen 4-35; Seite 9, Beispiel 1; Ansprüche 1,3,8 * <br> — — — — | 1,2,5-7 | |

Der vorliegende Recherchenbericht wurde für alle Patentansprüche erstellt

| Recherchenort | Abschlußdatum der Recherche | Prüfer |
|---|---|---|
| Den Haag | 22 April 91 | BOULOIS D.J-M. |